# EUROPEAN PATENT APPLICATION

(11) **EP 4 356 914 A1**
(43) Date of publication of application: **24.04.2024**
(21) Application number: 22824259.0
(22) Date of filing: 15.06.2022
(51) Int. Cl.: A61K 31/522, A61K 31/5377, C07D 473/32, C07D 471/04, C07D 401/12, C07D 401/14, A61P 35/00

(54) **IDH MUTANT INHIBITOR AND USE THEREOF**

(30) Priority: 15.06.2021 CN 202110661394
(71) Applicant: Wigen Biomedicine Technology (Shanghai) Co., Ltd., Shanghai 201203 (CN)
(72) Inventor: XIE, Yuli, Shanghai 201203 (CN); FAN, Houxing, Shanghai 201203 (CN); QIAN, Lihui, Shanghai 201203 (CN)
(74) Representative: Lipscombe, Martin John
(86) International application number: PCT/CN2022/099018
(87) International publication number: WO 2022/262784

(57) **Abstract**

Disclosed in the present invention are a class of IDH mutant inhibitors and use thereof. In particular, the present invention relates to a class of compounds of general formula (1), a method for preparing same, and use of the compound of general formula (1) or an optical isomer, a crystalline form or a pharmaceutically acceptable salt thereof as an irreversible inhibitor of IDH mutant in the preparation of an antitumor drug.

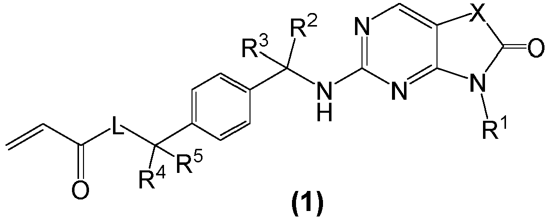

## Description

The present application claims priority to Chinese Application CN202110661394.5 filed on June 15, 2021, which is incorporated herein by reference in its entirety.

### TECHNICAL FIELD

The present invention relates to the field of pharmaceutical chemistry, and particularly to a class of novel IDH mutant inhibitors, a method for preparing same and use thereof.

### BACKGROUND

Isocitrate dehydrogenase (IDH) is an important enzyme involved in the tricarboxylic acid cycle. It catalyzes the conversion of isocitrate to α-ketoglutarate (α-KG), which is the rate-limiting step in the tricarboxylic acid cycle. There are three different isocitrate dehydrogenases in humans, IDH1, IDH2 and IDH3. IDH1 is mainly localized in the cytoplasm and peroxisomes, and IDH2 and IDH3 are mainly distributed in mitochondria.

IDH1 and IDH2 are the most common metabolic genes in the identification of human cancer gene mutations, and IDH mutations are found in low-grade gliomas, secondary malignant gliomas, melanoma, angioimmunoblastic T-cell lymphoma, myeloproliferative tumors, myelodysplastic syndromes (MDSs), and acute myelocytic leukemia (AML). IDH mutation sites in tumor cells are IDH1 Arg132 (R132), IDH2 Arg172 (R172) or IDH2 Arg140 (R140). These mutations result in a loss of functions of the wild-type IDH protein, and instead provide the ability to convert α-KG to a tumorigenic metabolite D-2-hydroxyglutarate (D-2HG). The tumorigenic metabolite 2-HG inhibits DNA or histone demethylases, resulting in hypermethylation of DNA and histone, thereby promoting the development of cancers. IDH inhibitors can reduce the *in vivo* tumorigenic metabolite D-2HG by inhibiting the activity of protein with IDH1/R132, IDH2/R172 or IDH2/R140 mutation, induce the demethylation of histone H3K9me3, and achieve the effect of inhibiting the development of tumors.

Therefore, targeting mutant IDH1 and IDH2 (mIDH1 and mIDH2) may be a promising approach for cancer therapy.

So far, some IDH small molecule inhibitors have been marketed, such as enasidenib and ivosidenib developed by Agios Pharmaceuticals Inc, both of which are noncovalent inhibitors. Covalent inhibitors of IDH have been reported by Eli Lilly and Company in Patent Nos. WO2017019429, WO2017213910 and WO2018111707, which have better selectivity for mutant IDH1 and IDH2 relative to wild-type IDH1 and IDH2. However, no IDH covalent inhibitors have entered clinical phase, and thus there is a need to study and explore the covalent IDH inhibitors with better activity and better druggability.

### SUMMARY

The present invention provides a compound of general formula (1) or an isomer, a crystalline form, a pharmaceutically acceptable salt, a hydrate or a solvate thereof: 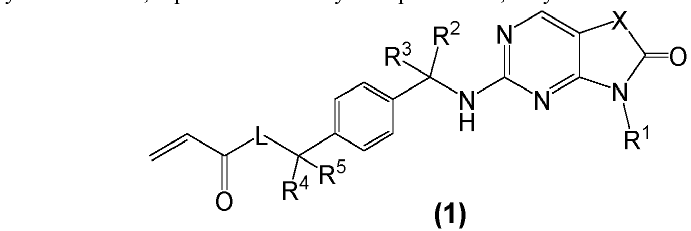 wherein in general formula (1):
L is 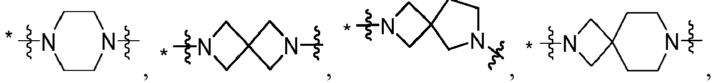 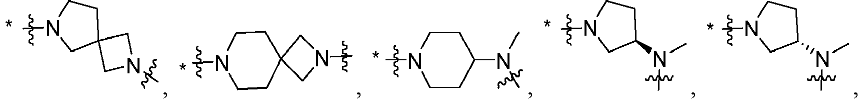 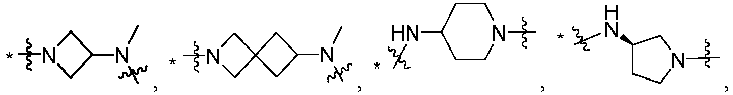 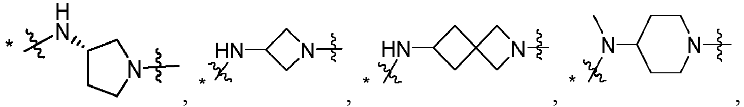 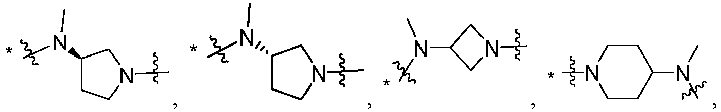 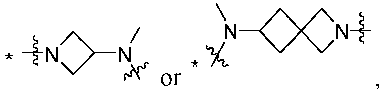 wherein "*" denotes a site linked to a carbonyl group;
X is NH or NMe;
R¹ is Me, Et, -CH₂CH₂CH₃, -CH(CH₃)₂, 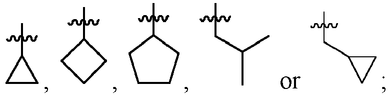
R² and R³ are independently H, Me or Et, or R² and R³, together with the carbon atom to which they are attached, form 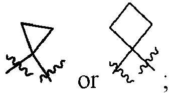
R⁴ and R⁵ are independently H, Me, Et, -CH₂CH₂CH₃, -CH(CH₃)₂, -CH₂CH(CH₃)₂, 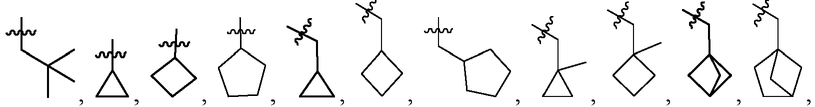 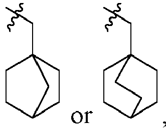 or R⁴ and R⁵, together with the carbon atom to which they are attached, form C3-C7 cycloalkyl, wherein the C3-C7 cycloalkyl may be substituted with halogen or C1-C3 alkyl.

In another preferred embodiment, in general formula (1), R² and R³ are independently H or Me, or R² and R³, together with the carbon atom to which they are attached, form

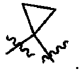

In another preferred embodiment, in general formula (1), R⁴ and R⁵ are independently H, Me, Et, -CH₂CH₂CH₃, -CH(CH₃)₂, -CH₂CH(CH₃)₂, 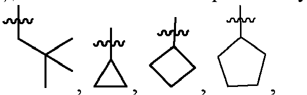 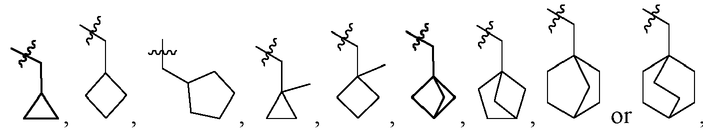 or R⁴ and R⁵, together with the carbon atom to which they are attached, form 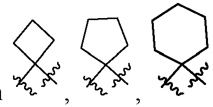 or

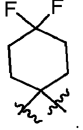

In another specific embodiment of the present invention, the compound of general formula (1) has one of the following structures: 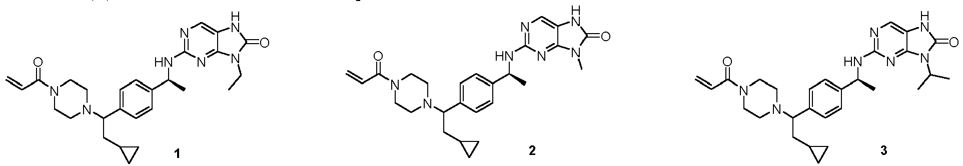 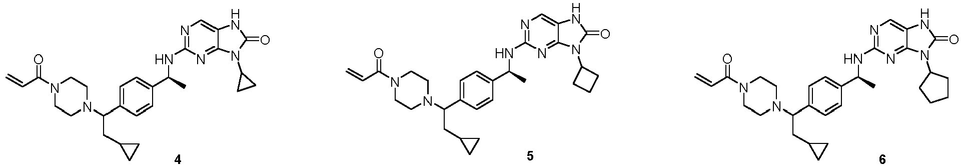 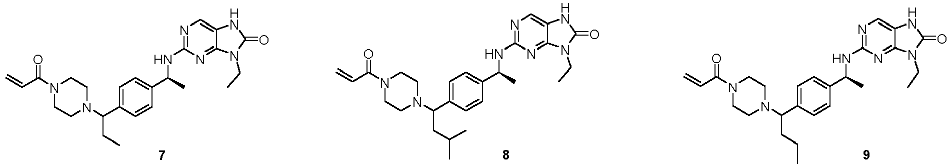 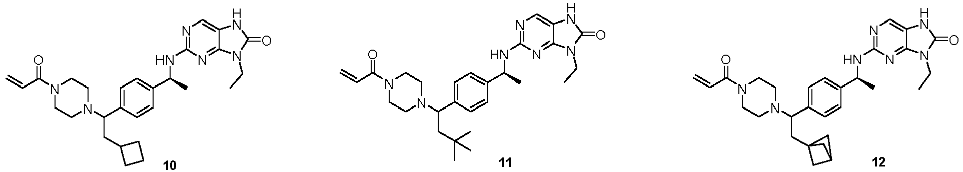 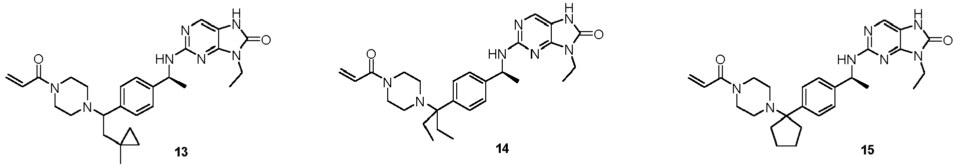 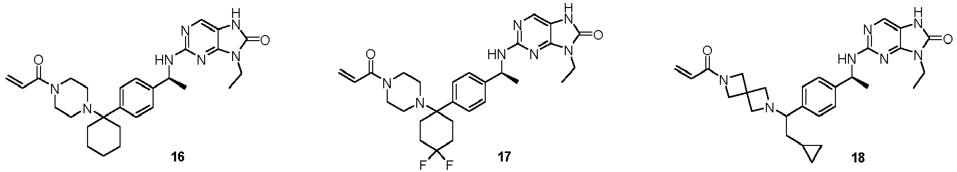 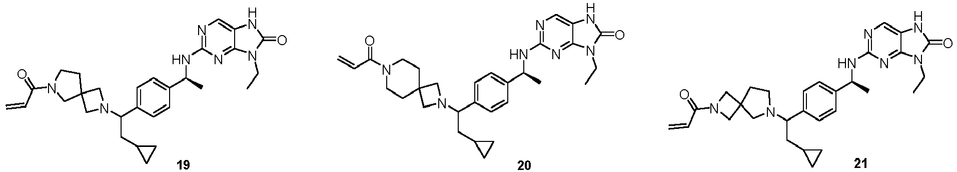 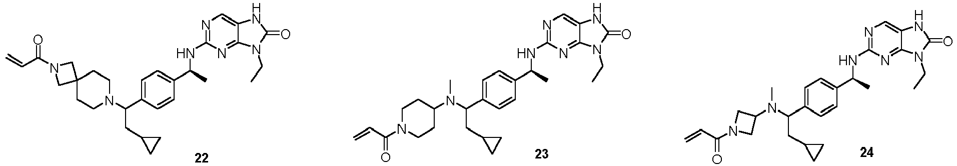 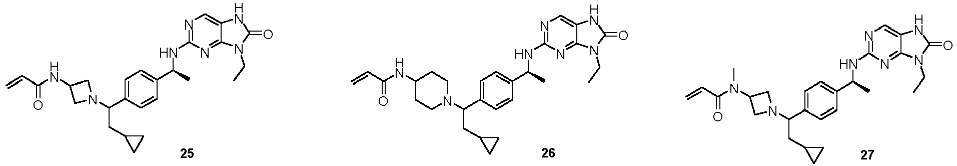 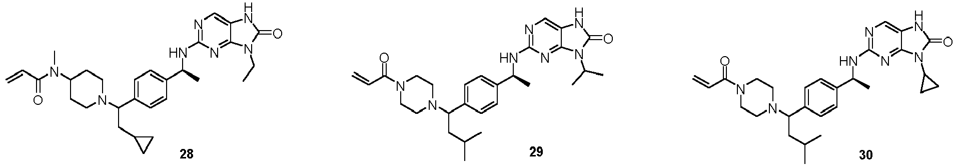 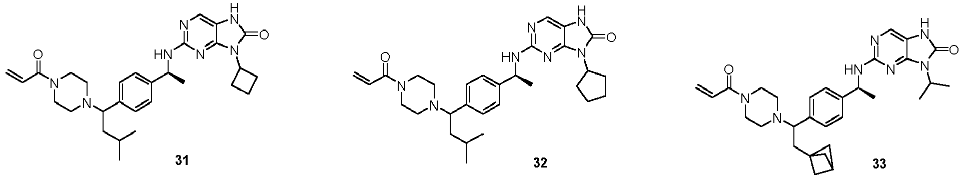 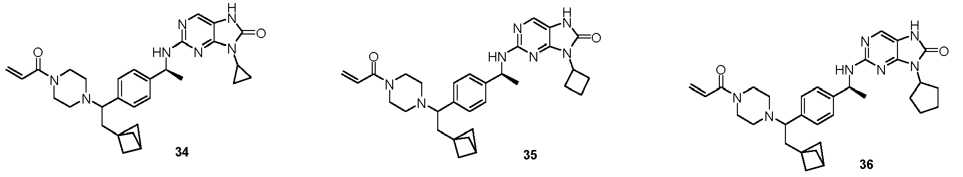 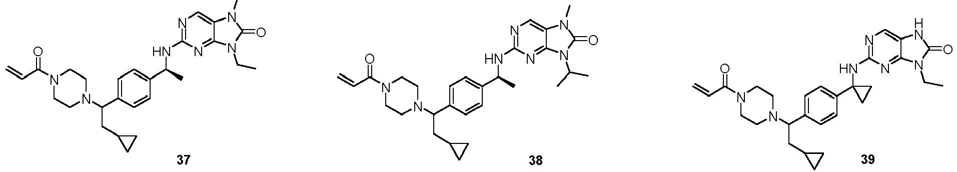 or

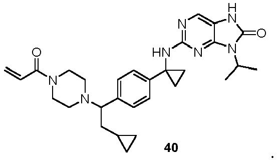

Another object of the present invention is to provide a pharmaceutical composition comprising a pharmaceutically acceptable carrier, diluent and/or excipient, and the compound of general formula (1) or the isomer, the crystalline form, the pharmaceutically acceptable salt, the hydrate or the solvate thereof of the present invention as an active ingredient.

Yet another object of the present invention is to provide use of the compound of general formula (1) or the isomer, the crystalline form, the pharmaceutically acceptable salt, the hydrate or the solvate thereof of the present invention, or the pharmaceutical composition described above in the preparation of a medicament for treating, regulating or preventing diseases associated with IDH mutant proteins.

It should be understood that both the above general description and the following detailed description of the present invention are exemplary and explanatory, and are intended to provide further explanation of the present invention claimed.

### Synthesis of Compounds

Methods for preparing the compounds of general formula (1) of the present invention are specifically described below, but these specific methods do not limit the present invention in any way.

The compounds of general formula (1) described above can be synthesized using standard synthetic techniques or well-known techniques in combination with the methods described herein. In addition, the solvents, temperatures and other reaction conditions mentioned herein may vary. Starting materials for the synthesis of the compounds can be obtained synthetically or commercially. The compounds described herein and other related compounds with different substituents can be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds can be modified by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compounds used, reaction temperature and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention further provides a method for preparing the compound of general formula (1), which is prepared using general reaction scheme 1 below:

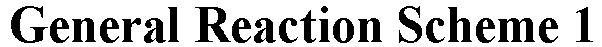

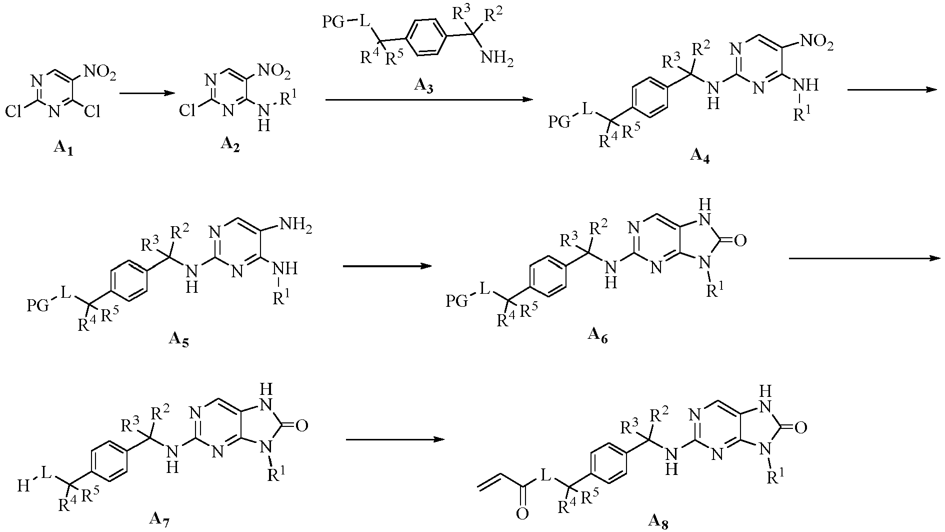

PG represents a protecting group for an amine group, and R¹, R², R³, R⁴, R⁵ and L are as defined above. As shown in general reaction scheme 1, starting material A1 is subjected to a substitution reaction to give compound A2; compounds A2 and A3 react under basic conditions to give compound A4; compound A4 is reduced at the nitro group to give compound A5; compound A5 is cyclized to give compound A6; the protecting group PG (e.g., Boc) in compound A6 is removed to give compound A7; and compound A7 reacts with acryloyl chloride to give the target compound A8.

### Further Forms of Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of general formula (1) to a reaction with acids, e.g., inorganic acids such as hydrochloric acid, hydrobromic acid, hydrofluoric acid, sulfuric acid, phosphoric acid, nitric acid, carbonic acid and the like; organic acids such as formic acid, acetic acid, propionic acid, oxalic acid, trifluoroacetic acid, malonic acid, succinic acid, fumaric acid, maleic acid, lactic acid, malic acid, tartaric acid, citric acid, picric acid, methanesulfonic acid, benzenesulfonic acid, p-toluenesulfonic acid and the like; and acidic amino acids such as aspartic acid, glutamic acid and the like.

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized and nanoparticle forms. In addition, the compound of general formula (1) includes crystalline forms, and may also be polymorphs. Polymorphs include different lattice arrangements of the same elements of a compound. The polymorphs generally have different X-ray diffraction spectra, infrared spectra, melting points, density, hardness, crystalline forms, optical and electrical properties, stability and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds. The compound of the present invention may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life and the like. All isotopic variations of the compound of the present invention, whether radioactive or not, are contained within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques and pharmacology are used. As used herein, "or" or "and" refers to "and/or" unless otherwise stated. Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, n-butyl, isobutyl, or tert-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu or ^{t}Bu.

Unless otherwise specified, "cycloalkyl" refers to a non-aromatic hydrocarbon ring system (monocyclic, bicyclic or polycyclic), and partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups and spiro rings (e.g., having 2, 3 or 4 fused rings). In some embodiments, cycloalkyl is monocyclic. In some embodiments, cycloalkyl is monocyclic or bicyclic. The ring carbon atoms of cycloalkyl may optionally be oxidized to form an oxo or sulfido group. Cycloalkyl further includes cycloalkylene. In some embodiments, cycloalkyl contains 0, 1 or 2 double bonds. In some embodiments, cycloalkyl contains 1 or 2 double bonds (partially unsaturated cycloalkyl). In some embodiments, cycloalkyl may be fused to aryl, heteroaryl, cycloalkyl and heterocycloalkyl. In some embodiments, cycloalkyl may be fused to aryl, cycloalkyl and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcarnyl, bicyclo[1.1.1]pentyl, bicyclo[2.1.1]hexyl and the like.

Unless otherwise specified, "halogen" (or halo) refers to fluorine, chlorine, bromine or iodine. The term "halo" (or "halogenated") before a group name indicates that the group is partially or fully halogenated, that is, substituted in any combination with F, Cl, Br or I, preferably with F or Cl.

"Optional" or "optionally" means that the subsequently described event or circumstance may, but does not necessarily, occur, and the description includes instances where the event or circumstance occurs and instances where the event or circumstance does not occur.

The substituent "-O-CH₂-O-" means that two oxygen atoms in the substituent are linked to two adjacent carbon atoms in the heterocycloalkyl, aryl or heteroaryl, for example

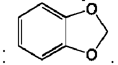

When the number of a linker group is 0, such as -(CH₂)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl and thiopyranyl are six-membered rings, and cyclopentyl, pyrrolyl, furanyl and thienyl are five-membered rings.

The term "moiety" refers to a specific portion or functional group of a molecule. A chemical moiety is generally considered to be a chemical entity contained in or attached to a molecule.

Unless otherwise stated, the absolute configuration of a stereogenic center is represented by a wedged solid bond ( 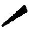 ) and a wedged dashed bond ( 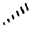 ), and the relative configuration of a stereogenic center is represented by a straight solid bond ( 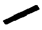 ) and a straight dashed bond ( 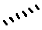 ). A wavy line ( 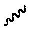 ) represents a wedged solid bond ( 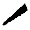 ) or a wedged dashed bond ( 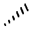 ), or a wavy line ( 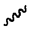 ) represents a straight solid bond ( 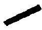 ) or a straight dashed bond ( 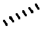 ).

Unless otherwise stated, a single bond or a double bond is represented by -̅ -̅ -̅.

### Specific Pharmaceutical and Medical Terminology

The term "acceptable", as used herein, means that a formulation component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present invention may contain one or more asymmetric centers (chiral center or axial chirality) and thus occur in the form of a racemate, a racemic mixture, a single enantiomer, a diastereomeric compound and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present invention. The present invention is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual value is within a particular value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides a method for treating a disease, including but not limited to a condition (e.g., cancer) associated with an IDH mutant protein, with the compound of general formula (1) or the pharmaceutical composition of the present invention.

In some embodiments, a method for treating cancer is provided, the method including administering to an individual in need thereof an effective amount of any aforementioned pharmaceutical composition including the compound of structural general formula (1). In some embodiments, the cancer is mediated by an IDH mutant protein. In other embodiments, the cancer is a hematologic cancer and a solid tumor, including but not limited to, leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma or all cancer metastases.

### Route of Administration

The compound and the pharmaceutically acceptable salt thereof of the present invention can be made into various formulations including a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious adverse effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills, and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present invention include ointments, pulvises, patches, sprays, and inhalants. The active ingredient is mixed under sterile conditions with a physiologically acceptable carrier and any preservatives, buffers or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient and the like will also be considered, which are well known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific examples for reference only. After reading the description of the present invention, those skilled in the art can make various changes or modifications to the present invention, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present invention: CDCl₃ represents deuterated chloroform; DCM represents dichloromethane; dioxane represents 1,4-dioxane; DIPEA represents diisopropylethylamine; DMSO represents dimethyl sulfoxide; EA represents ethyl acetate; EtOH represents ethanol; h represents hour; H₂ represents hydrogen; KOH represents potassium hydroxide; LC-MS represents liquid chromatography-mass spectrometry; min represents minute; mL represents milliliter; MS represents mass spectrometry; n-BuLi represents n-butylaluminum; NaBH(OAc)₃ represents sodium triacetoxyborohydride; NH₄Cl represents ammonium chloride; NMR represents nuclear magnetic resonance; Pd/C represents palladium on carbon; PE represents petroleum ether; THF represents tetrahydrofuran; Ti(Oⁱ⁻Pr)₄ represents titanium tetraisopropoxide.

### Preparation Example 1. 2-Chloro-N-ethyl-5-nitropyrimidin-4-amine

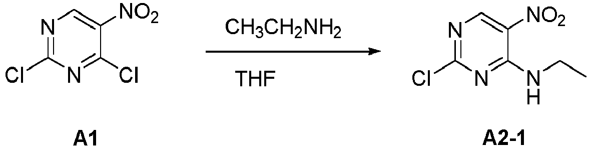

2,4-Dichloro-5-nitropyrimidine (2 g, 10.31 mmol) was dissolved in THF (30 mL). Under argon atmosphere, aqueous ethylamine solution (1.33 g, 70%) was added dropwise at -70 °C. After dropwise addition, the mixed solution was warmed to room temperature and reacted for about 0.5 h. After the completion of the reaction as detected by LC-MS, water (50 mL) was added, and the mixed solution was extracted twice with EA (30 mL × 2). The organic phases were combined and concentrated. The residue was purified by column chromatography to give a yellow solid product A2-1 (1.763 g, 84% yield), ESI-MS m/z: 203 [M+H]⁺.

Target intermediates **A2-2 to A2-6** were obtained using different starting materials according to the synthesis method for the intermediate **A2-1**.

**Table 1. Structural formulas of intermediates A-2 to A-6**

| **Intermediate** | **Compound structure** | **MS [M+H]⁺** | **Intermediate** | **Compound structure** | **MS [M+H]⁺** |
|---|---|---|---|---|---|
| A2-2 | 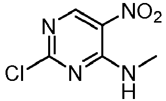 | 189 | A2-3 | 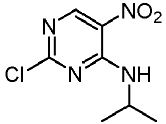 | 217 |
| A2-4 | 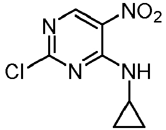 | 215 | A2-5 | 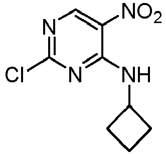 | 229 |
| A2-6 | 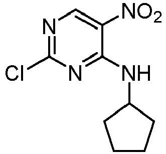 | 243 | | | |

### Preparation Example 2. Synthesis of tert-butyl 4-(1-(4-((S)-1-aminoethyl)phenyl)-2-cyclopropylethyl)piperazine-1-carboxylate (A3-1)

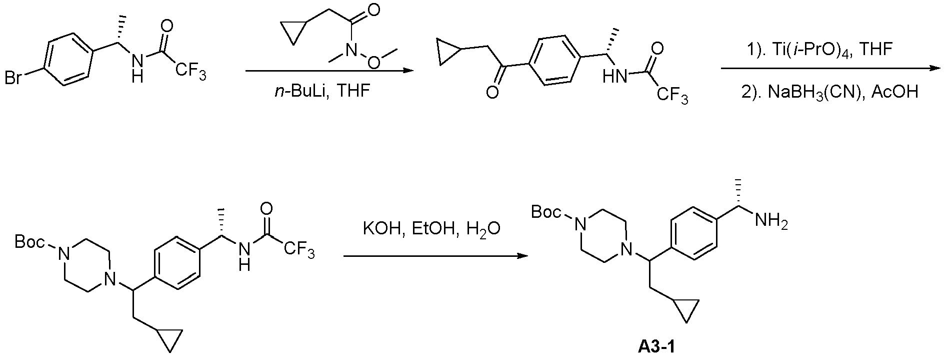

### Synthesis of (S)-N-(1-(4-(2-cyclopropylacetyl)phenyl)ethyl)-2,2,2-trifluoroacetamide

(*S*)-*N*-(1-(4-bromophenyl)ethyl)-2,2,2-trifluoroacetamide (10.7 g, 36.2 mmol) was dissolved in anhydrous THF (100 mL), and *n*-BuLi (2.5 M, 30 mL, 72.3 mmol) was added dropwise at -78 °C under argon atmosphere. After the addition was completed, the mixed solution was incubated at -78 °C to -60 °C for about 1 h for reaction, and then a solution of 2-cyclopropyl-*N*-methoxy-*N*-methylacetamide (5.7 g, 39.8 mmol) in anhydrous THF (50 mL) was slowly added dropwise. After dropwise addition, the mixed solution was incubated at a constant temperature for another 0.5 h for reaction. After the completion of the reaction as detected by LC-MS, the mixed solution was quenched with a saturated aqueous NH₄Cl solution (100 mL), and extracted twice with EA (50 mL × 2). The organic phase was concentrated. The residue was purified by column chromatography to give a white solid product (6.06 g, 56% yield), ESI-MS m/z: 300 [M+H]⁺.

### Synthesis of tert-butyl 4-(2-cyclopropyl-1-(4-((S)-1-(2,2,2-trifluoroacetylamino)et hyl)phenyl)ethyl)piperazine-1-carboxylate

(*S*)-*N*-(1-(4-(2-cyclopropylacetyl)phenyl)ethyl)-2,2,2-trifluoroacetamide (3.068 g, 10.25 mmol) and *tert-*butyl piperazine-1-carboxylate (3.82 g, 20.5 mmol) were dissolved in anhydrous THF (50 mL), and Ti(*i*-PrO)₄ (15 mL, 51.25 mmol) was added dropwise under argon atmosphere. The mixed solution reacted overnight at 60 °C, and then cooled to room temperature. MeOH (20 mL) and NaBH(OAc)₃ (1.288 g, 20.5 mmol) were added. The mixture reacted at room temperature for 10 h. A small amount of starting material remained as detected by LC-MS. The mixed solution was quenched with water (100 mL), and extracted with EA (50 mL × 2). The organic phase was concentrated, and the residue was purified by column chromatography to give a white solid product (957 mg, 20% yield) while recovering the unreacted starting materials (2.2 g), ESI-MS m/z: 470 [M+H]⁺.

### Synthesis of tert-butyl 4-(1-(4-((S)-1-aminoethyl)phenyl)-2-cyclopropylethyl)piperazine-1-carboxylate (A3-1)

*tert*-Butyl 4-(2-cyclopropyl-1-(4-((*S*)-1-(2,2,2-trifluoroacetylamino)ethyl)phenyl)ethyl) piperazine-1-carboxylate (3.74 g, 7.97 mmol) was dissolved in EtOH/H₂O (100 mL/20 mL), KOH (2.24 g, 39.87 mmol) was added in batches in an ice bath, and the mixed solution was heated to 50 °C under argon atmosphere and reacted for 3 h. After the completion of the reaction as detected by LC-MS, the mixed solution was concentrated at reduced pressure to remain about 30 mL. The remaining mixture was diluted with water (50 mL), and extracted with DCM (50 mL × 2). The organic phases were combined, washed with saturated sodium chloride solution (50 mL), dried over anhydrous sodium sulfate, filtered, and concentrated at reduced pressure to give the target compound **A3-1** (3.854 g, 100% yield), ESI-MS m/z: 374 [M+H]⁺.

Target intermediates **A3-2 to A3-20** were obtained using different starting materials according to the synthesis method for the intermediate **A3-1.**

**Table 2. Structural formulas of intermediates A3-2 to A3-20**

| **Int ermediate** | **Compound structure** | **MS [M+H]⁺** | **Int ermediate** | **Compound structure** | **MS [M+H]⁺** |
|---|---|---|---|---|---|
| **A3-2** | 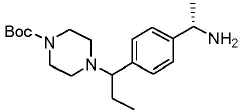 | 348 | **A3-3** | 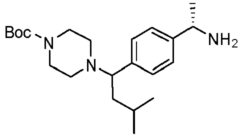 | 376 |
| **A3-4** | 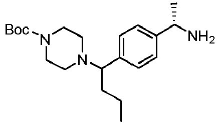 | 362 | **A3-5** | 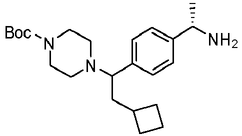 | 388 |
| **A3-6** | 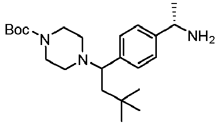 | 390 | **A3-7** | 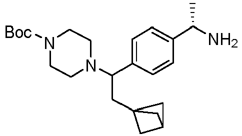 | 400 |
| **A3-8** | 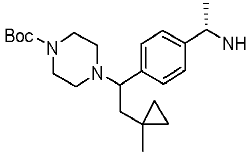 | 388 | **A3-9** | 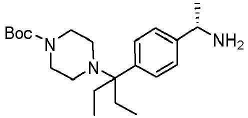 | 376 |
| **A3-10** | 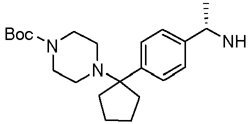 | 374 | **A3-11** | 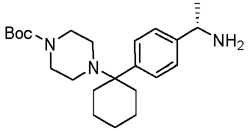 | 388 |
| **A3-12** | 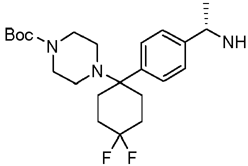 | 424 | **A3-13** | 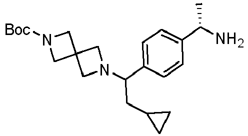 | 386 |
| **A3-14** | 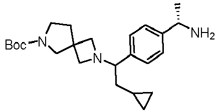 | 400 | **A3-15** | 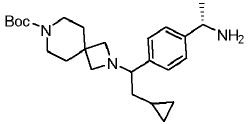 | 414 |
| **A3-16** | 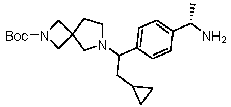 | 400 | **A3-17** | 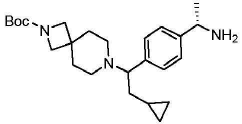 | 414 |
| **A3-18** | 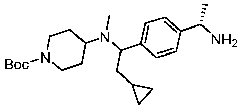 | 402 | **A3-19** | 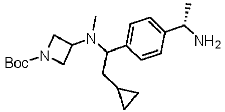 | 374 |
| **A3-20** | 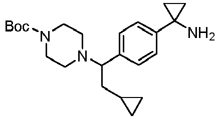 | 386 | | | |

### Example 1. Synthesis of 2-(((1S)-1-(4-(1-(4-acryloylpiperazin-1-yl)-2-cyclopropylethyl)phenyl)ethyl)amino)-9-ethyl-7,9-dihydro-8H-purin-8-one

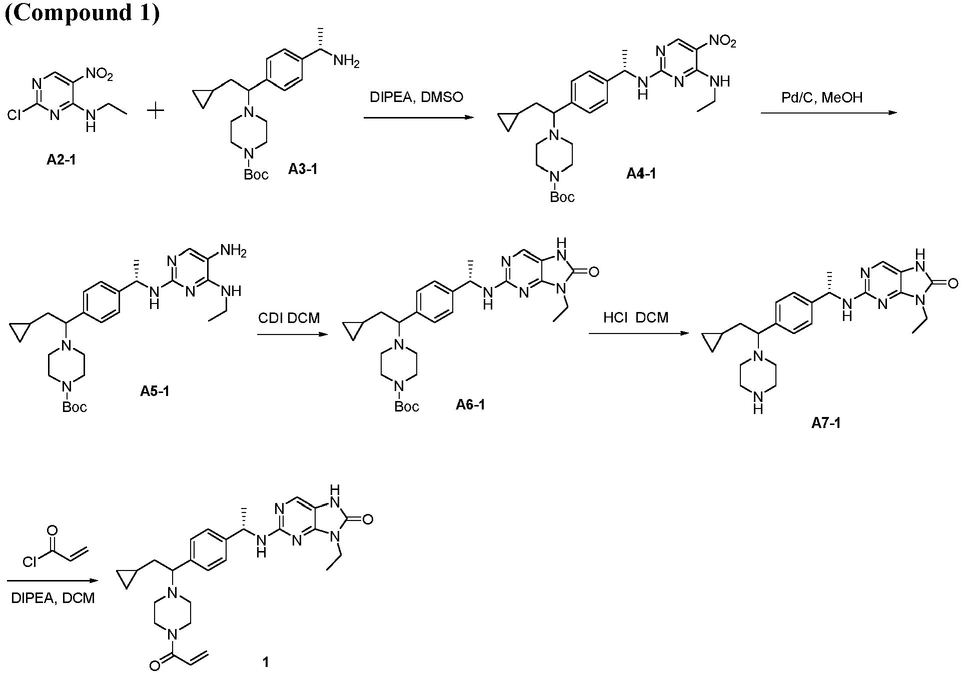

### Step 1: Synthesis of compound A4-1

Compound **A3-1** (200 mg, 0.535 mmol) and compound A2-1 (130 mg, 0.642 mmol) were dissolved in DMSO (5 mL). DIPEA (207 mg, 1.605 mmol) was added. The mixed solution reacted at 80 °C for about 3 h. After the completion of the reaction as detected by LC-MS, water (30 mL) was added, and the mixed solution was extracted with EA (20 mL × 2). The organic phases were combined and concentrated. The residue was purified by column chromatography to give a yellow solid product **A4-1** (290 mg, 91% yield), ESI-MS m/z: 540 [M+H]⁺.

### Step 2: Synthesis of compound A5-1

Compound **A4-1** (290 mg, 0.537 mmol) was dissolved in MeOH (10 mL), and Pd/C (40 mg, 10%) was added. The mixed solution reacted overnight at room temperature after being purged with hydrogen. After the completion of the reaction as detected by LC-MS, the mixed solution was filtered and concentrated to give a purple solid **A5-1** (236 mg, 86% yield), ESI-MS m/z: 510 [M+H]⁺.

### Step 3: Synthesis of compound A6-1

Compound **A5-1** (236 mg, 0.463 mmol) was dissolved in DCM (10 mL), and CDI (150 mg, 0.926 mmol) was added. The mixed solution reacted overnight at room temperature. After the completion of the reaction as detected by LC-MS, the mixed solution was concentrated at reduced pressure. The residue was purified by Flash to give a dark purple solid **A6-1** (151 mg, 61% yield), ESI-MS m/z: 536 [M+H]⁺.

### Step 4: Synthesis of compound A7-1

Compound **A6-1** (151 mg, 0.282 mmol) was dissolved in DCM (5 mL), 4 M HCl/Diox (1 mL, 4 mmol) was added, and the mixed solution was stirred and reacted at room temperature for about 3 h. After the completion of the reaction as detected by LC-MS, the mixed solution was concentrated at reduced pressure to give a yellow solid product **A7-1** (170 mg, 100% yield), ESI-MS m/z: 436 [M+H]⁺.

### Step 5: Synthesis of compound 1

Compound **A7-1** (170 mg, 0.282 mmol) and DIPEA (183 mg, 1.41 mmol) were dissolved in DCM (5 mL). The mixed solution was cooled to 0 °C in an ice bath under argon atmosphere. A solution of acryloyl chloride (23 mg, 0.254 mmol) in DCM (3 mL) was added dropwise. The mixed solution reacted in an ice bath for about 10 min. After the completion of the reaction as detected by LC-MS, water (10 mL) was added, followed by stirring and liquid separation, and then the aqueous phase was extracted with DCM (10 mL). The organic phases were combined. The concentrated residue was purified by pre-TLC to give a pale yellow solid product (100 mg, 72% yield). ¹H NMR (400 MHz, CDCl₃) δ: 7.81 (s, 1H), 7.32 (d, *J=* 7.9 Hz, 2H), 7.18 (d, *J =* 8.0 Hz, 2H), 6.49 (dd, *J =* 16.9, 10.5 Hz, 1H), 6.23 (dd, *J =* 16.8, 1.9 Hz, 1H), 5.64 (dd, *J* = 10.5, 1.9 Hz, 1H), 5.26 (s, 1H), 5.10 (p, *J=* 6.9 Hz, 1H), 3.86 (q, *J=* 7.2 Hz, 2H), 3.63 (m, 2H), 3.49 (m, 2H), 3.44-3.36 (m, 1H), 2.38 (m, 4H), 1.89-1.79 (m, 1H), 1.65 (m, 2H), 1.55 (d, *J=* 6.8 Hz, 3H), 1.29-1.25 (m, 3H), 0.40 (q, *J=* 6.4, 4.8 Hz, 1H), 0.36-0.24 (m, 2H), -0.01-0.09 (m, 2H); ESI-MS m/z: 490 [M+H]⁺.

### Examples 2-40. Synthesis of compounds 2-40

By the procedures similar to those in the synthesis of compound 1, the target compounds **2-40** in Table 3 may be obtained using different intermediates as starting materials.

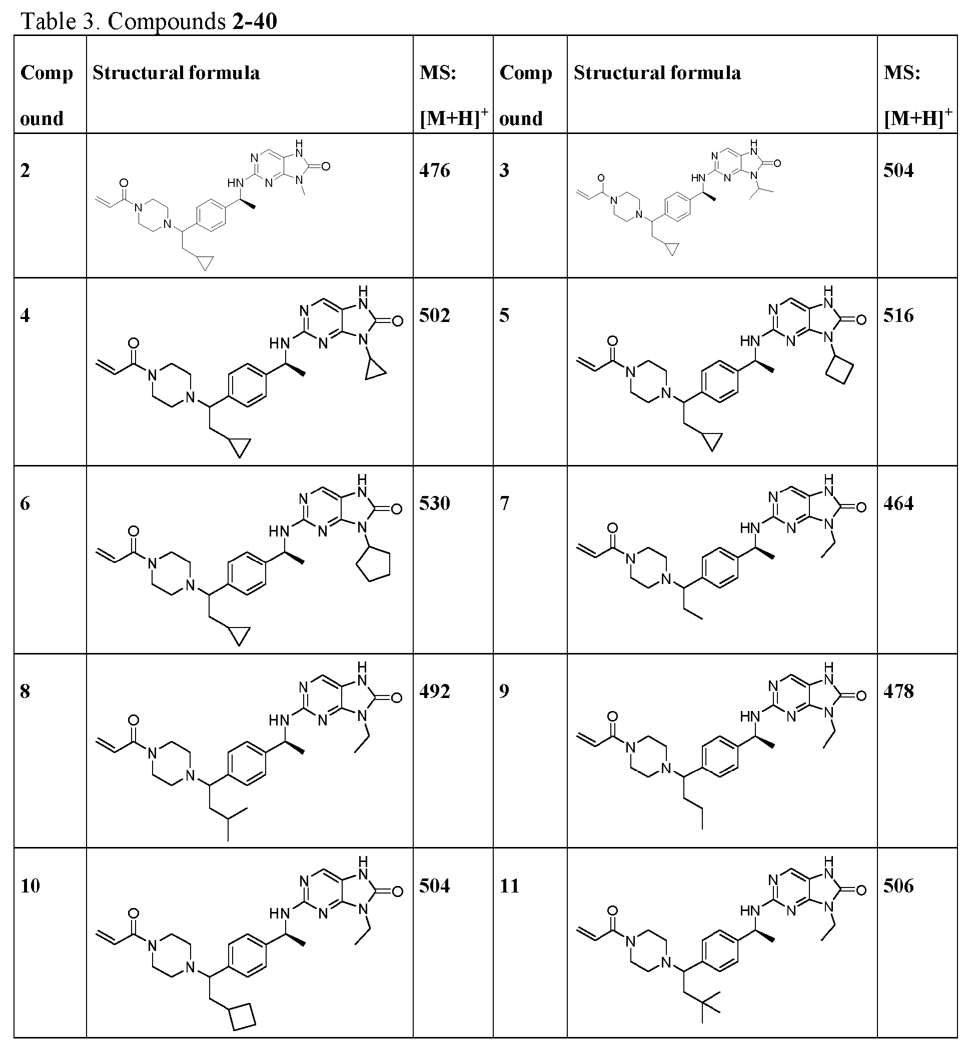

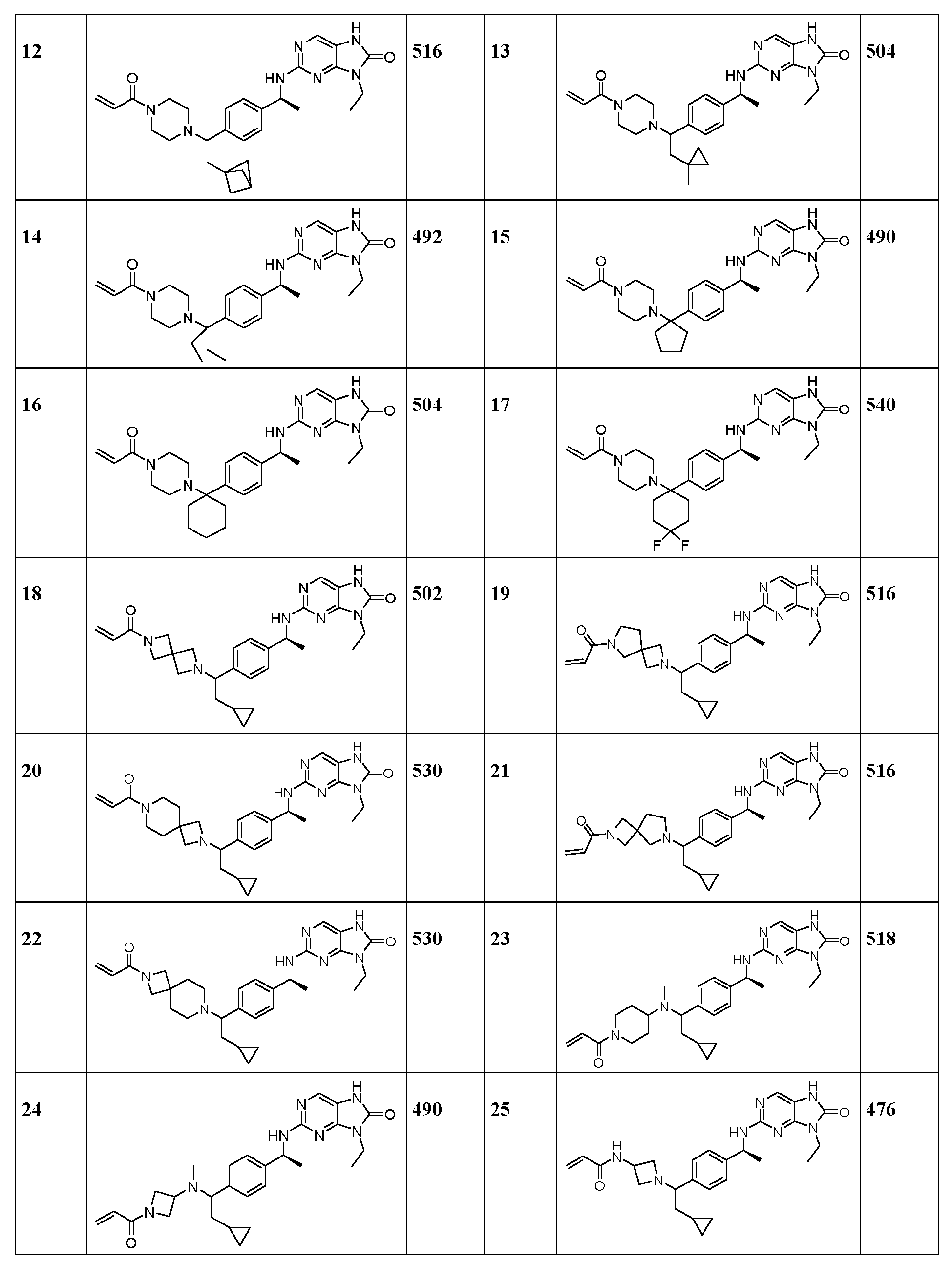

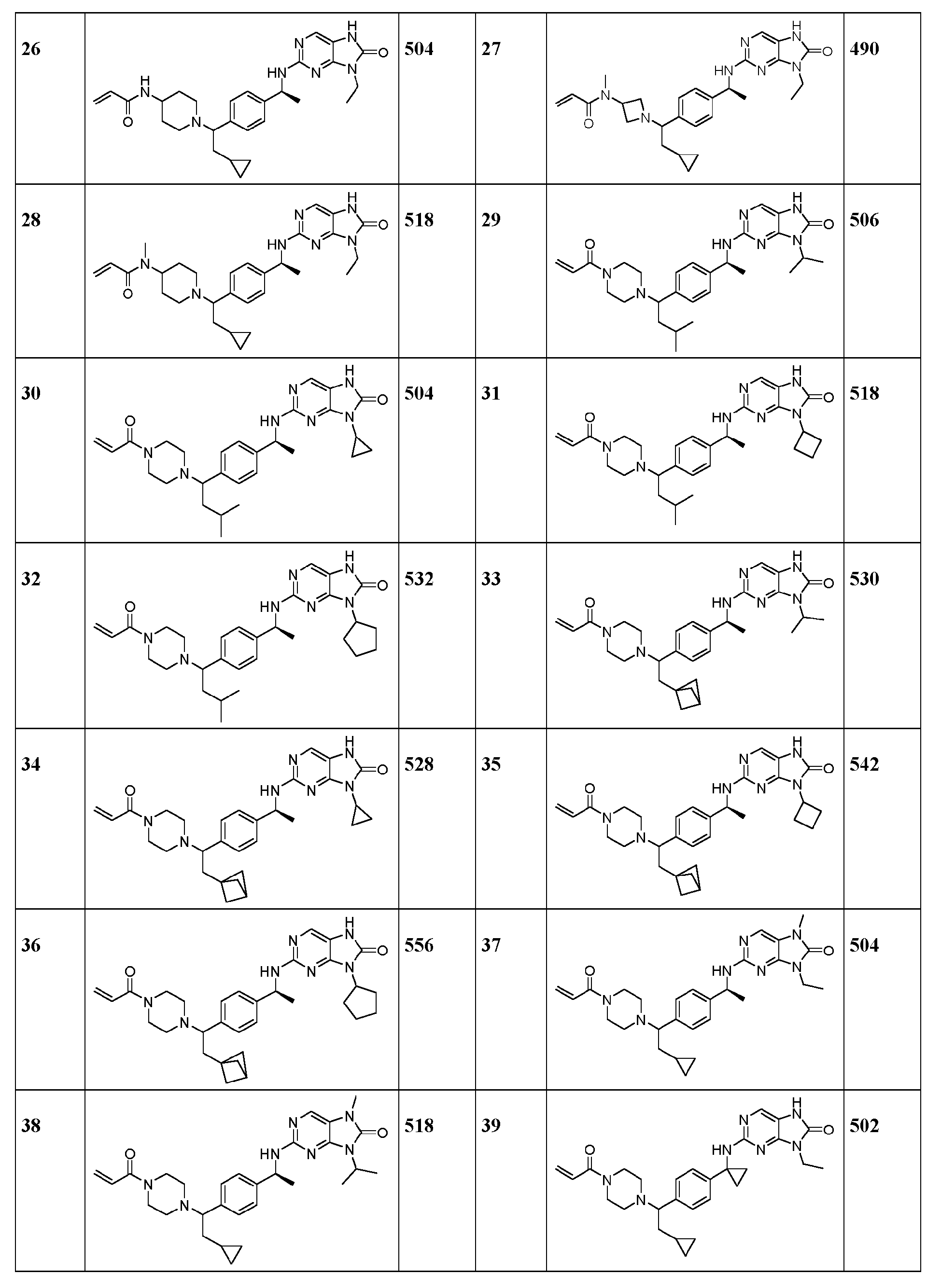

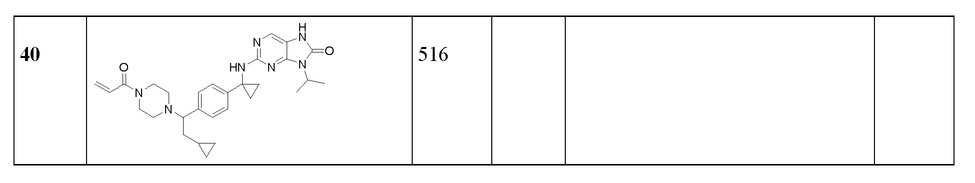

### Example 41. Preparation of Chiral Isomers of Compound of the Present Invention

The compound of the present invention comprises one or more chiral centers. Various optically pure isomers of the compound of the present invention may be prepared by using optically pure intermediates as starting materials. Alternatively, optically pure isomers of the compound of the present invention may be prepared by using chiral HPLC or achiral HPLC.

Compound **1** of the present invention may be obtained as two optically pure isomers **1-1** and **1-2** of the compound **1** by using the method described above:

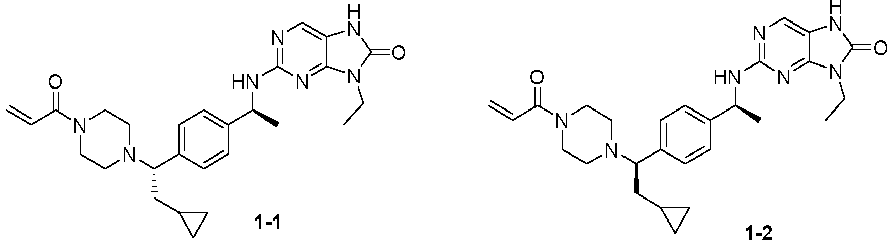

Compounds **3, 4, 5, 12, 29, 30, 33 and 34** were chirally resolved by the same synthesis or preparation method to give chiral isomer pairs **3-1/3-2, 4-1/4-2, 5-1/5-2, 12-1/12-2, 29-1/29-2, 30-1/30-2, 33- 1/33-2 and 34-1/34-2,** respectively:

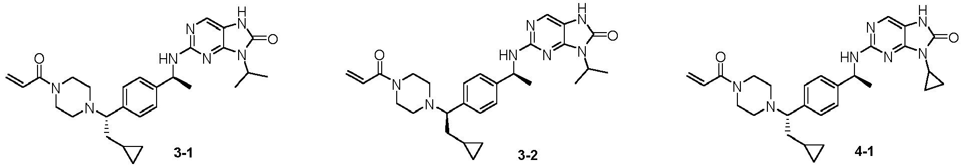

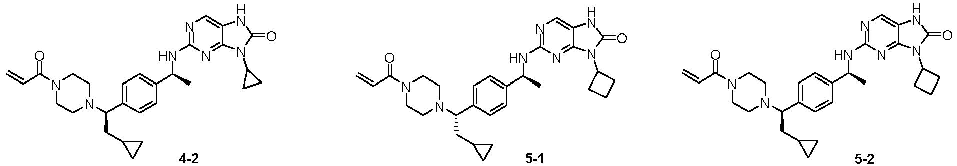

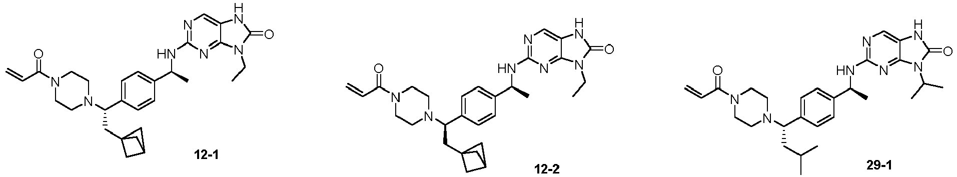

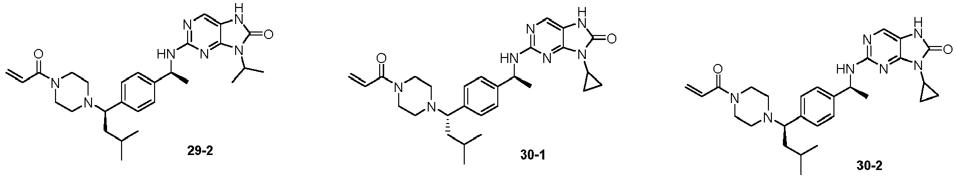

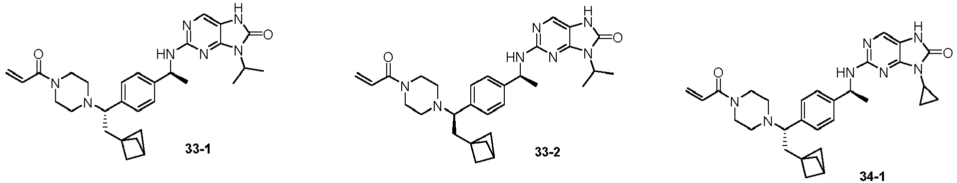

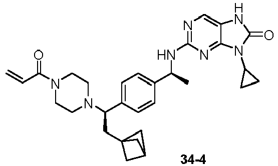

Other compounds of the present invention may also be prepared by similar synthesis or preparation methods to give their corresponding chiral isomers.

**Table 4. NMR data of some compounds of the present invention**

| **Compound** | **Nuclear magnetic hydrogen spectrum** |
|---|---|
| **3-1** | ¹H NMR (400 MHz, Chloroform-*d*) δ 9.36 (s, 1H), 7.81 (s, 1H), 7.31 (d, *J =* 7.6 Hz, 2H), 7.17 (d, *J* = 7.9 Hz, 2H), 6.48 (dd, *J* = 16.8, 10.5 Hz, 1H), 6.22 (dd, *J =* 16.8, 2.0 Hz, 1H), 5.63 (dd, *J =* 10.5, 2.0 Hz, 1H), 5.36 (d, *J* = 7.0 Hz, 1H), 5.02 (p, *J=* 7.1 Hz, 1H), 4.57 (p, *J =* 6.9 Hz, 1H), 3.63 (s, 2H), 3.48 (s, 2H), 3.38 (dd, *J =* 9.4, 5.0 Hz, 1H), 2.39 (d, *J =* 26.9 Hz, 4H), 1.85 (td, *J=* 11.3, 9.0, 5.2 Hz, 1H), 1.53 (dd, *J* = 8.9, 6.9 Hz, 6H), 1.48 (d, *J =* 15.0 Hz, 1H), 1.31 (d, *J* = 6.9 Hz, 3H), 0.38 (dd, *J =* 7.9, 4.9 Hz, 1H), 0.29 (tt, *J =* 7.5, 2.2 Hz, 2H), -0.05 (ddd, *J=* 13.9, 8.4, 3.5 Hz, 2H). |
| **3-2** | ¹H NMR (400 MHz, cdcl₃) δ 8.77 (s, 1H), 7.80 (s, 1H), 7.32 (d, *J* = 8.1 Hz, 2H), 7.17 (d, *J* = 8.1 Hz, 2H), 6.48 (dd, *J* = 16.8, 10.5 Hz, 1H), 6.22 (dd, *J* = 16.8, 1.9 Hz, 1H), 5.63 (dd, *J* = 10.6, 1.9 Hz, 1H), 5.28 (d, *J* = 7.0 Hz, 1H), 5.03 (p, *J* = 6.8 Hz, 1H), 4.57 (h, *J* = 7.0 Hz, 1H), 3.63 (s, 2H), 3.49 (s, 2H), 3.40 (dd, *J =* 9.3, 5.1 Hz, 1H), 2.48 - 2.32 (m, 4H), 1.85 (ddd, *J* = 14.2, 9.3, 5.4 Hz, 1H), 1.54 (dd, *J* = 9.2, 6.9 Hz, 6H), 1.34 (d, *J* = 6.9 Hz, 3H), 0.46 - 0.36 (m, 1H), 0.36 - 0.23 (m, 2H), -0.06 (ddt, *J* = 13.6, 9.1, 4.1 Hz, 2H). |
| **4-1** | ¹H NMR (400 MHz, Chloroform-*d*) δ 8.79 (s, 1H), 7.80 (s, 1H), 7.32 (d, *J* = 7.9 Hz, 2H), 7.18 (d, *J* = 7.9 Hz, 2H), 6.49 (dd, *J* = 16.8, 10.5 Hz, 1H), 6.22 (dd, *J* = 16.8, 2.0 Hz, 1H), 5.64 (dd, *J* = 10.6, 1.9 Hz, 1H), 5.30 (d, *J* = 5.8 Hz, 1H), 5.07 (p, *J* = 6.9 Hz, 1H), 3.64 (s, 2H), 3.50 (s, 2H), 3.40 (dd, *J* = 9.3, 5.1 Hz, 1H), 2.90 - 2.83 (m, 1H), 2.37 (d, *J* = 13.5 Hz, 4H), 1.84 (td, *J* = 9.0, 4.7 Hz, 1H), 1.58 - 1.54 (m, 3H), 1.54 - 1.49 (m, 1H), 1.06 - 0.96 (m, 4H), 0.42 (dd, *J* = 8.0, 5.1 Hz, 1H), 0.30 (ddq, *J* = 13.0, 8.8, 5.1, 4.5 Hz, 2H), -0.05 (ddd, *J* = 13.1, 7.9, 4.7 Hz, 2H). |
| **5-1** | ¹H NMR (400 MHz, Chloroform-*d*) δ 8.83 (s, 1H), 7.81 (s, 1H), 7.33 (d, *J* = 7.9 Hz, 2H), 7.18 (d, *J* = 7.9 Hz, 2H), 6.48 (dd, *J* = 16.8, 10.5 Hz, 1H), 6.23 (dd, *J* = 16.8, 1.9 Hz, 1H), 5.64 (dd, *J* = 10.5, 1.9 Hz, 1H), 5.38 (d, *J* = 6.6 Hz, 1H), 5.03 (p, *J* = 6.9 Hz, 1H), 4.78 (p, *J* = 8.9 Hz, 1H), 3.63 (s, 2H), 3.49 (s, 2H), 3.38 (dt, *J* = 7.4, 3.6 Hz, 1H), 3.12 (p, *J* = 10.1 Hz, 1H), 2.69 (p, *J* = 10.0 Hz, 1H), 2.48 - 2.32 (m, 4H), 2.26 - 2.17 (m, 1H), 2.00 (s, 1H), 1.89 - 1.81 (m, 2H), 1.72 (s, 1H), 1.56 (d, *J* = 6.9 Hz, 3H), 1.53 - 1.47 (m, 1H), 0.42 - 0.33 (m, 1H), 0.33 - 0.23 (m, 2H), - 0.05 (ddd, *J* = 17.3, 9.3, 3.0 Hz, 2H). |
| **6** | ¹H NMR (400 MHz, Chloroform-*d*) δ 9.09 (s, 1H), 7.80 (s, 1H), 7.32 - 7.27 (m, 2H), 7.17 (d, *J =* 7.9 Hz, 2H), 6.48 (dd, *J =* 16.8, 10.6 Hz, 1H), 6.26 - 6.17 (m, 1H), 5.63 (dd, *J =* 10.6, 1.9 Hz, 1H), 5.35 (t, *J* = 6.2 Hz, 1H), 5.02 (dt, *J* = 7.9, 4.0 Hz, 1H), 4.66 (qt, *J* = 8.7, 4.3 Hz, 1H), 3.64 (d, *J =* 10.0 Hz, 2H), 3.48(m, 2H), 3.38 (dt, *J* = 9.6, 4.9 Hz, 1H), 2.37 (m, 4H), 2.27 - 2.17 (m, 1H), 2.03 - 1.80 (m, 5H), 1.76 - 1.59 (m, 4H), 1.54 (d, *J* = 6.8 Hz, 4H), 0.44 - 0.23 (m, 3H), - 0.07 (dd, *J* = 8.6, 4.1 Hz, 1H). |

### Example 42. Detection of 2-HG in Supernatant of U87-IDH1 R132H Cells

U87MG cells overexpressing the mIDH1 R132H mutation or HT1080 cells carrying IDH1 R132C were seeded in a 48-well plate and a 96-well plate at 50,000 cells/well and 10,000 cells/well, respectively. After the cells were incubated overnight for adhesion, the supernatants were removed. The cell culture media containing serially diluted compounds were added, and the cells were incubated for 72 h. After 72 h, the culture media were collected and diluted 10-fold and 20-fold with water, respectively. Acetonitrile was added to extract the metabolites. The 2-HG content in the culture media was analyzed by LC-MS-MS. The percentage inhibition and IC₅₀ of the compounds on 2-HG in the supernatant were calculated as compared with the control group.

**Table 5. Inhibition rate of some compounds of the present invention on 2-HG in the supernatant of U87-IDH R132H cells**

| Compound | Inhibition rate | | | Compound | Inhibition rate | | |
|---|---|---|---|---|---|---|---|
| | 100 nM | 10 nM | 1 nM | | 100 nM | 10 nM | 1 nM |
| **1** | A | A | B | **2** | A | B | C |
| **3** | A | A | C | **4** | A | A | C |
| **5** | A | A | C | **6** | A | B | D |
| **7** | A | B | C | **8** | A | A | C |
| **9** | A | B | C | **10** | A | B | C |
| **11** | A | B | C | **12** | A | A | C |
| **13** | A | B | C | **14** | A | A | C |
| **15** | A | A | C | **16** | A | B | C |
| **17** | A | B | C | **18** | A | A | C |
| **19** | A | B | C | **20** | A | B | C |
| **21** | A | B | C | **22** | A | B | B |
| **23** | A | B | C | **24** | A | B | C |
| **25** | A | B | B | **26** | A | B | B |
| **27** | A | B | D | **28** | A | B | C |
| **29** | A | A | B | **30** | A | A | C |
| **31** | A | A | C | **32** | A | A | C |
| **33** | A | A | B | **34** | A | A | B |
| **35** | A | A | C | **36** | A | A | C |
| **37** | B | B | D | **38** | B | B | D |
| **39** | A | B | D | **40** | A | B | D |
| **Ivosidenib** | B | D | D | **LY-3410738** | A | A | C |

| | | | | | | | |
|---|---|---|---|---|---|---|---|
| A indicates an inhibition rate greater than 90% B indicates an inhibition rate greater than 60% but less than or equal to 90% C indicates an inhibition rate greater than 30% but less than or equal to 60% D indicates an inhibition rate less than or equal to 30% | | | | | | | |

**Table 6. Inhibition rate of some compounds of the present invention on 2-HG in the supernatant of HT1080 cells**

| Compound | **IC₅₀ (nM)** | Compound | **IC₅₀ (nM)** | Compound | **IC₅₀ (nM)** |
|---|---|---|---|---|---|
| **LY-3410738** | 1.90 | 1 | 4.48 | 1-1 | 2.24 |
| 3 | 2.06 | 3-1 | 1.22 | 3-2 | 2.36 |
| 4-1 | 11.5 | 5-1 | 2.63 | 6-1 | 3.22 |

### Example 43. Stability Test of Human Liver Microsomes and Mouse Liver

### Microsomes

After the 1 µM compound was incubated with 500 µg/ml of human or mouse liver microsomes and a NADPH regeneration system at 37 °C for different times, respectively, LC-MS-MS was used to analyze the remaining amount of the compound and calculate T_{1/2}.

**Table 7. Liver microsomal stability of some compounds of the present invention**

| Compound | **T_{1/2} (min)** | |
|---|---|---|
| | Human | Mouse |
| **LY-3410738** | 1.80 | 1.60 |
| 1-1 | 11.3 | 4.4 |
| 3-1 | 5.0 | 4.0 |
| 6-1 | 4.4 | 3.9 |

### Example 44. Detection of 2-HG in Tumor Tissues

Nude mice were inoculated subcutaneously with 1 × 10⁶ HT1080 cells. When the tumor volume reached 100-150 mm³, the mice were randomly grouped into a solvent control group and a group of 20 mg/kg compound 1, 3, 29, 33, or LY-3410738. The tumors were collected three days and seven days after continuous administration, weighed, digested with a digestion solution and then homogenized. The levels of 2-HG in the tumor tissues were determined by LC-MS-MS. The percentage inhibition of compounds on 2-HG in tumor tissues was calculated as compared with the control group.

**Table 8. Inhibition rate of compounds on 2-HG in HT1080 tumor tissues**

| **Compound** | **1** | **3** | **29** | **33** | **LY-3410738** |
|---|---|---|---|---|---|
| 3 days | 49±18% | 58±3% | 45±38% | 57±1.8% | 43±11% |
| 7 days | 94±0.5% | 97±1% | 92±4% | 96±0.4% | 85±12% |

Activity data in Tables 5-8 demonstrate that the compounds of general formula (1) of the present invention have stronger inhibitory activity against the level of 2-HG in the supernatants of U87-IDH R132H cells and HT1080 cells and in tumor tissues compared with the marketed IDH inhibitor ivosidenib, indicating that they have stronger ability to inhibit IDH1 R132H and IDH1 R132C mutant proteins. Meanwhile, these compounds have similar or stronger activity compared with LY-3410738 (compound 2 in Patent No. WO2018111707).

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A compound of general formula (1) or an isomer, a crystalline form, a pharmaceutically acceptable salt, a hydrate or a solvate thereof: 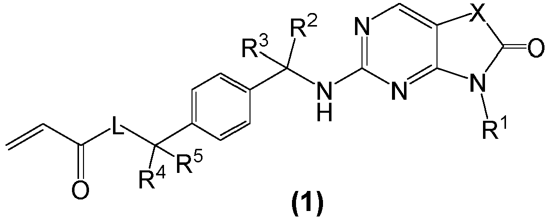 wherein in general formula (1):
L is 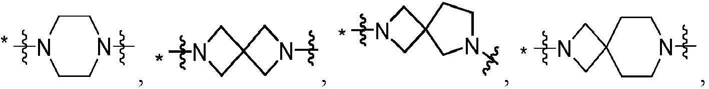 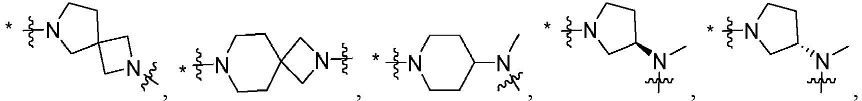 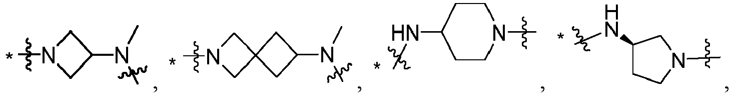 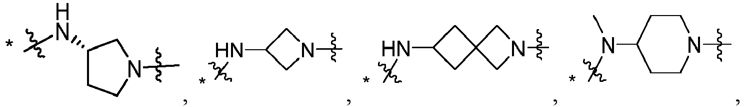 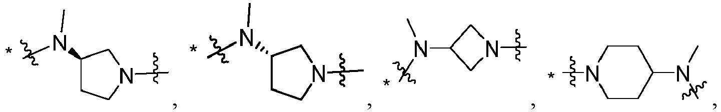 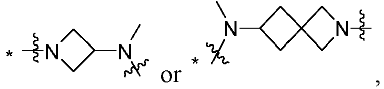 wherein "*" denotes a site linked to a carbonyl group;
X is NH or NMe
R¹ is Me, Et, -CH₂CH₂CH₃, -CH(CH₃)₂, 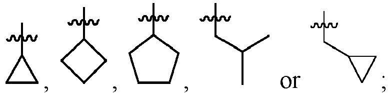
R² and R³ are each independently H, Me or Et, or R² and R³, together with the carbon atom to which they are attached, form 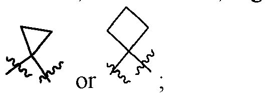
R⁴ and R⁵ are each independently H, Me, Et, -CH₂CH₂CH₃, -CH(CH₃)₂, - CH₂CH(CH₃)₂, 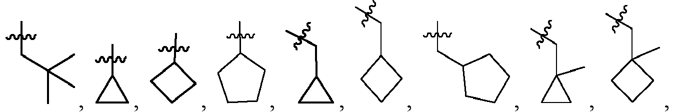 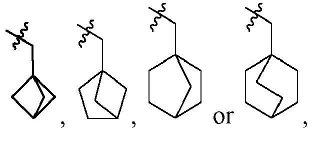 or R⁴ and R⁵, together with the carbon atom to which they are attached, form C3-C7 cycloalkyl, wherein the C3-C7 cycloalkyl may be substituted with halogen or C1-C3 alkyl.

2. The compound or the isomer, the crystalline form, the pharmaceutically acceptable salt, the hydrate or the solvate thereof according to claim 1, wherein in general formula (1), R² and R³ are independently H or Me, or R² and R³. together with the carbon atom to which they are attached, form

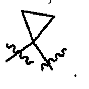

3. The compound or the isomer, the crystalline form, the pharmaceutically acceptable salt, the hydrate or the solvate thereof according to claim 1, wherein in general formula (1), R⁴ and R⁵ are independently H, Me, Et, -CH₂CH₂CH₃, -CH(CH₃)₂, - CH₂CH(CH₃)₂ 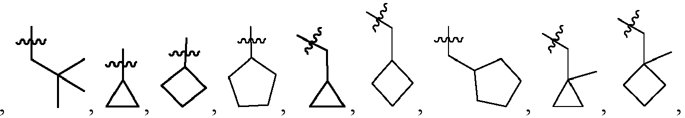 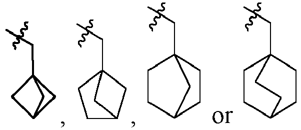 ; or R⁴ and R⁵, together with the carbon atom to which they are attached, form

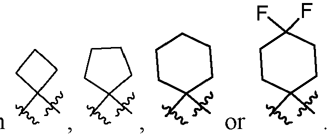

4. The compound or the isomer, the crystalline form, the pharmaceutically acceptable salt, the hydrate or the solvate thereof according to any one of claims 1-3, wherein the compound has one of the following structures: 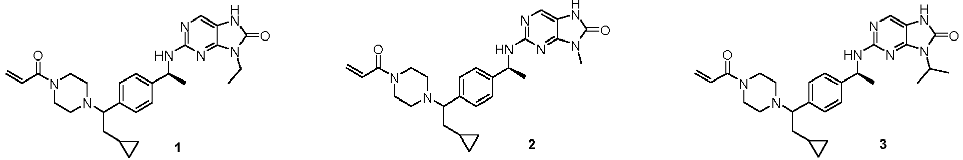 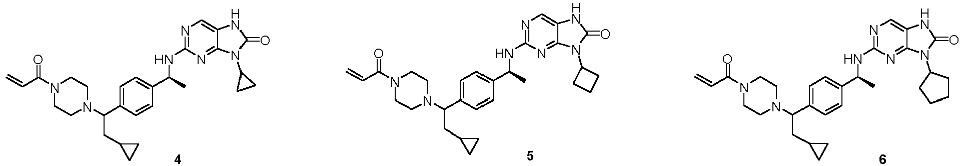 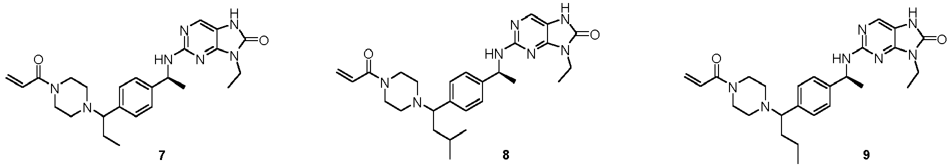 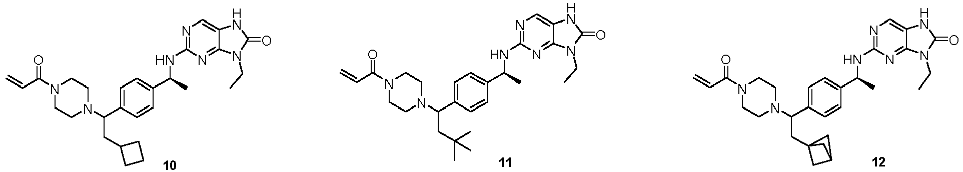 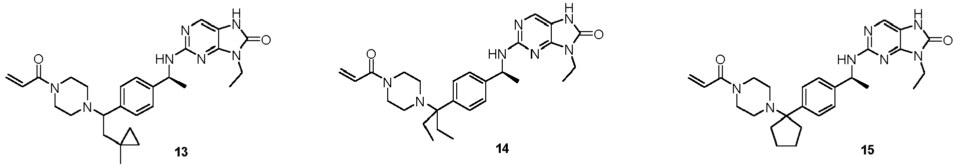 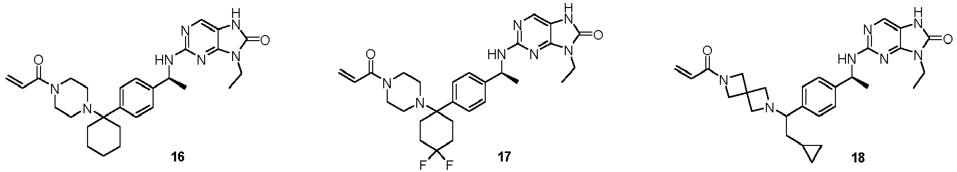 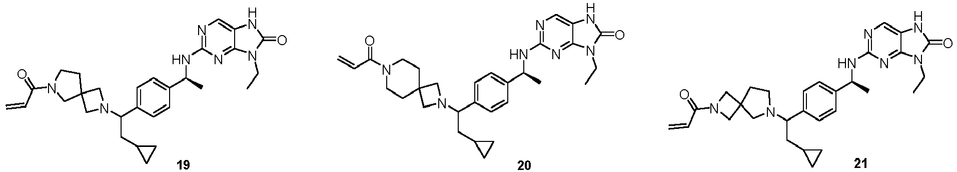 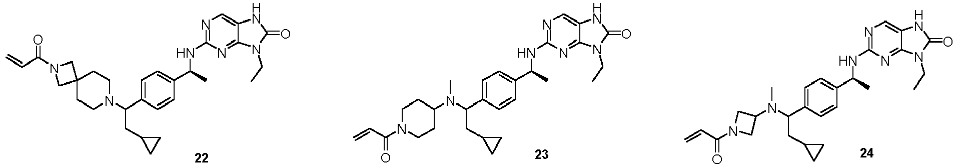 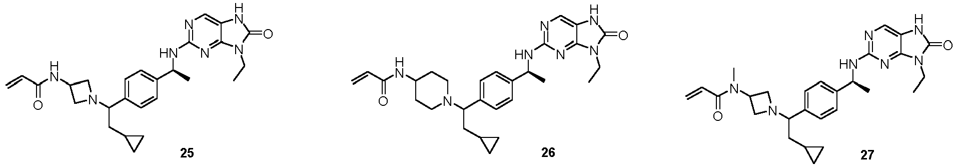 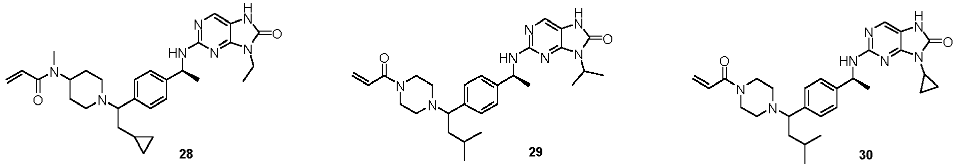 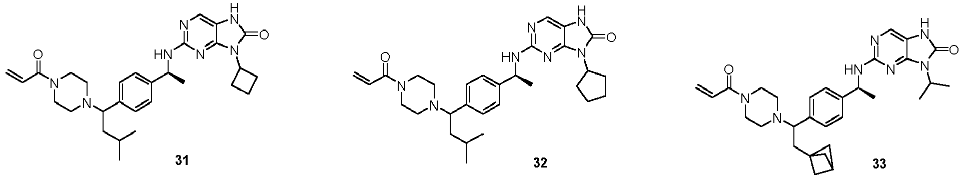 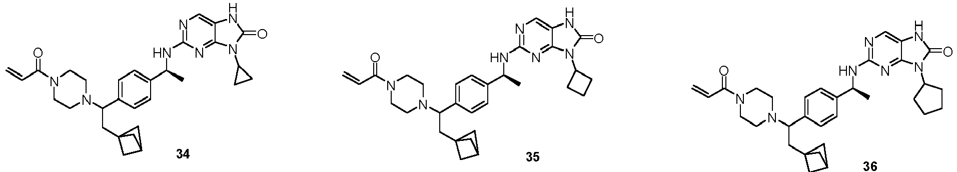 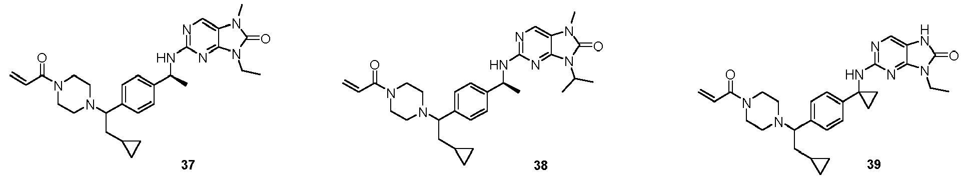 or

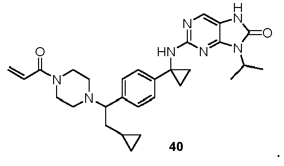

5. A pharmaceutical composition, comprising a pharmaceutically acceptable excipient or carrier; and the compound or the isomer, the crystalline form, the pharmaceutically acceptable salt, the hydrate or the solvate thereof according to any one of claims 1-4 as an active ingredient.

6. Use of the compound or the isomer, the crystalline form, the pharmaceutically acceptable salt, the hydrate or the solvate thereof according to any one of claims 1-4, or the pharmaceutical composition according to claim 5 in the preparation of a medicament for treating a related disease mediated by an IDH mutant protein.
